# EUROPEAN PATENT APPLICATION

(11) **EP 0 547 772 A1**
(43) Date of publication of application: **23.06.1993**
(21) Application number: 92310534.0
(22) Date of filing: 18.11.1992
(51) Int. Cl.: A61B 17/39, A61B 17/34

(54) **Laparoscopic instrument**

(30) Priority: 16.12.1991 US 808429
(71) Applicant: DEXIDE, INC., Fort Worth Texas 76118-6995 (US)
(72) Inventor: Freitas, Michael W., Texas 76180 (US); Podell, Randy J., Georgia 30062 (US)
(74) Representative: Jackson, Peter Arthur

(57) **Abstract**

A laparoscopic electrosurgical instrument and probe has an instrument head (105) carried on an electrode (104) and is selectively moveable between extended and retracted positions to permit the device to be used as a probe when the electrode is completely retracted. A fluid passageway (111) also is provided for irrigation/suction purposes.

## Description

Laparoscopic surgical procedures gain access to the interior of the anatomical cavity by first using an implement, such as a trocar spike, cannula or a needle having a sharpened point, to pierce or puncture the bodily tissues, muscles, membranes, or the like, which may form a portion of or surround the cavity wall.

Similarly, in many laparoscopic procedures, a small incision may be made in the skin of the patient along the abdomen, for example, and the sharp point of a larger penetrating implement, such as a trocar spike of suitable length and diameter, may be inserted into the incision, and pushed until the point punctures the cavity wall. A sleeve accompanies the implement into the puncture wound to serve as a lining for preserving the shape of the passageway created by the implement and for the insertion of the endoscope, laparoscope, or the like to view and operate upon organs within the cavity. The cavity is typically insufflated with an inert gas during all surgical procedures.

In some endoscopic procedures, it has been found necessary to incorporate an electrosurgical procedure which uses a device which incorporates blades, needles, and the like, which are electrically activated by an electrical circuit which generates a radio frequency current with differentwave form shapes to optimize tissue cutting, hemostasis, and the like. A hand piece is configured to receive the working electrode by which the radio frequency currents can be applied to the patient's tissue for cutting or hemostasis. Such electrosurgical devices have been utilized in laparoscopy in which the hand piece is carefully inserted through the trocar sleeve into the abdominal cavity. Typical of such electrosurgical apparatuses is that as disclosed in US-A-4,754,754.

In many instances, it is desirable to "probe", manipulate, or move organs, bodily tissue, muscles, membranes and the like, within the abdominal cavity during endoscopic surgery, prior to or subsequent to the electrosurgical proceeding. In such instance, it has been found disadvantageous to require withdrawal of the electrosurgical instrument from within the abdominal wall and reinsertion therein of a second or auxiliary probing instrument. Because of the sharp end or point of the instrument head portion of most electrosurgical instruments utilized in endoscopic surgery, such instruments are not satisfactory for the "probing" procedure, as described, because the sharp edge or pointed end could accidently cut or otherwise damage such body components.

US-A-5,035,695, discloses an electrosurgical instrument with a switch to selectively activate the electric current therethrough, in combination with a passageway for irrigation/suction purposes. Such device can not be satisfactorily utilized as a probe because the instrument head, or cutting edge or outer tip always is exposed beyond the housing of the device, thus making its use as probe, in endoscopic procedures, or otherwise, disadvantageous.

The present invention provides a combination laparoscopic electrosurgical instrument and probe. The combination laparoscopic electrosurgical instrument and probe are introducible into a trocar assembly by an operator, i.e., a surgeon, into the abdominal cavity of a patient. The instrument and probe comprise an elongate body having forward and rearward ends. Electrode means are housed within the body for supplying electrical or radio frequency signal extending substantially from the rearward end to near the forward end. Means, such as a carriage, are provided within the body and extend to the electrode means for selective positioning thereof between rearward-most retracted and forward-most extended positions along a slidable path within the body relative to the forward end. Shifting means are provided which are disposed on the body and are operatively attached to the carriage means for selectively moving the electrode means between retracted and extended positions. An instrument head is secured to the electrode means near the forward end of the body and is selectively extendable through the forward end when the electrode means is in the extended position and is selectively retracted within the body when the electrode means is in the retracted position, whereby, when in the retracted position, the instrument may function as a surgical probe. A fluid passageway is disposed in the instrument and extends from the forward end of the body for transmitting suction or irrigation fluids between the abdominal cavity and the apparatus. Preferably, the fluid passageway will be disposed within the housing and concentric to the electrode means, with the electrode means being disposed within the fluid passageway. Preferably, the apparatus will further include insulation means to electrically insulate the operator and patient from the laparoscopic instrument.

Sealing means are also disposed within the body forsealing between the body and the electrode means to prevent fluid communication thereacross, with, preferably, the sealing means including a conically shaped elastomeric element which is disposed around the electrode means, the conically shaped elastomeric having a smaller diameter portion facing toward the forward end and extending to a larger outer diameter portion facing toward the rearward end.

A port extends through the body and into the passageway, with the port being toward the forward end of the body relative to the sealing means. The port receives one of a liquid or gaseous transmitting conduit. The pressure of the fluid through the port and the passageway enhances the sealing engagement of the sealing means between the electrode means and the body.

The combination electrosurgical instrument and probe are introducable into the abdominal cavity through a trocar. Preferably, the trocar will have a sleeve with the first end extending into the abdominal cavity through a laparoscopic incision in the abdominal wall, the first end of the sleeve having a first external dimension passage through the incision. Means are provided for expanding a portion of the first end of the sleeve within the abdomen so that the external dimension of the first end of the sleeve within the abdomen is expanded to a larger, second external dimension. The first end of the sleeve abuts the inner abdominal wall about the incision when expanded to the second external dimension to resist withdrawal of the trocar assembly from the abdominal cavity. A passageway is provided through the assembly and interior of the sleeve for introduction or removal of the electrosurgical instrument and probe relative to the abdomen.

In the accompanying drawings:
Fig. 1 is a partial longitudinal cross-sectional view of the instrument of combined electrosurgical device and probe introduced within a trocar, the head of the device being in retracted position.
Fig. 2 is a view similar to that of Fig. 1, showing the instrument within the trocar and interior of an abdominal wall, as in surgery, with the head of the instrument in expanded position.
Fig. 3 is a cross-sectional view taken along line 3-3 of Fig. 4, illustrating the opening and alignment means of the outer body of the electrosurgical device, for alignment of the instrument head therein.
Fig. 4 is a perspective view of the instrument, with the instrument head in expanded position.
Fig. 5 is a cross-sectional view of the instrument, with the instrument head in retracted position.
Fig. 6 is a view similar to that of Fig. 5, showing the instrument head in expanded position.
Fig. 7 is a view similar to Figs. 5 and 6, illustrating an alternative embodiment.

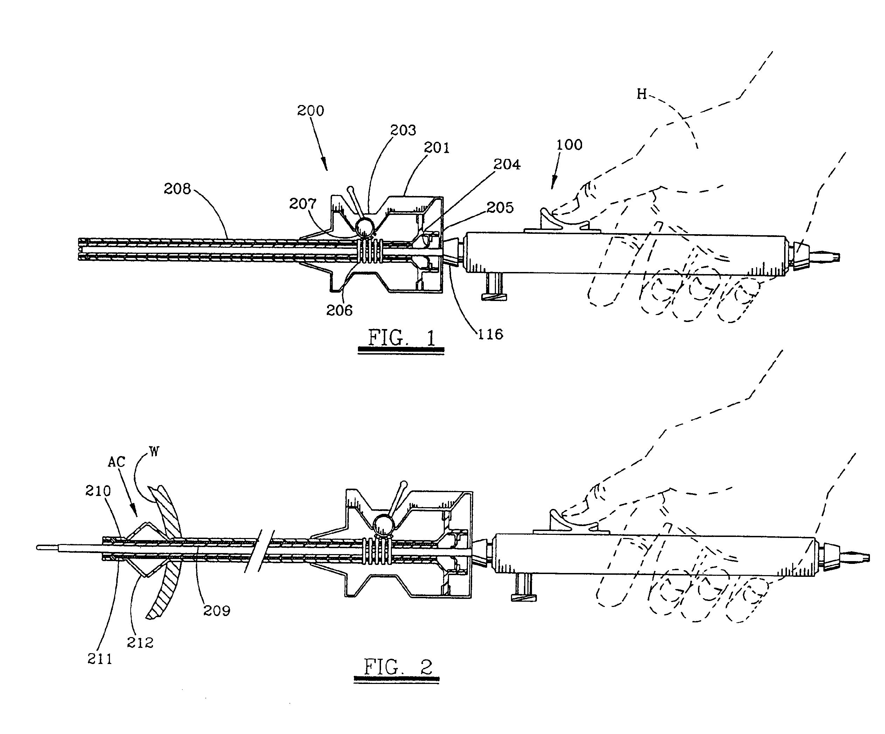

Now, with first reference to Fig. 1, there is shown the electrosurgical instrument and probe 100 introduced within a trocar 200. The electrosurgical instrument and probe 100 may be inserted into the trocar 200 and, thereafter, the combination trocar 200 and instrument and probe 100 may be introduced to the abdominal wall W and into the abdominal cavity AC, as in Fig. 2. Alternatively, the trocar 200 may first be introduced through the abdominal wall W and into the abdominal cavity AC, and, thereafter, the electrosurgical instrument and probe 100 may be introduced into the outer-most end of the trocar 200, as described below.

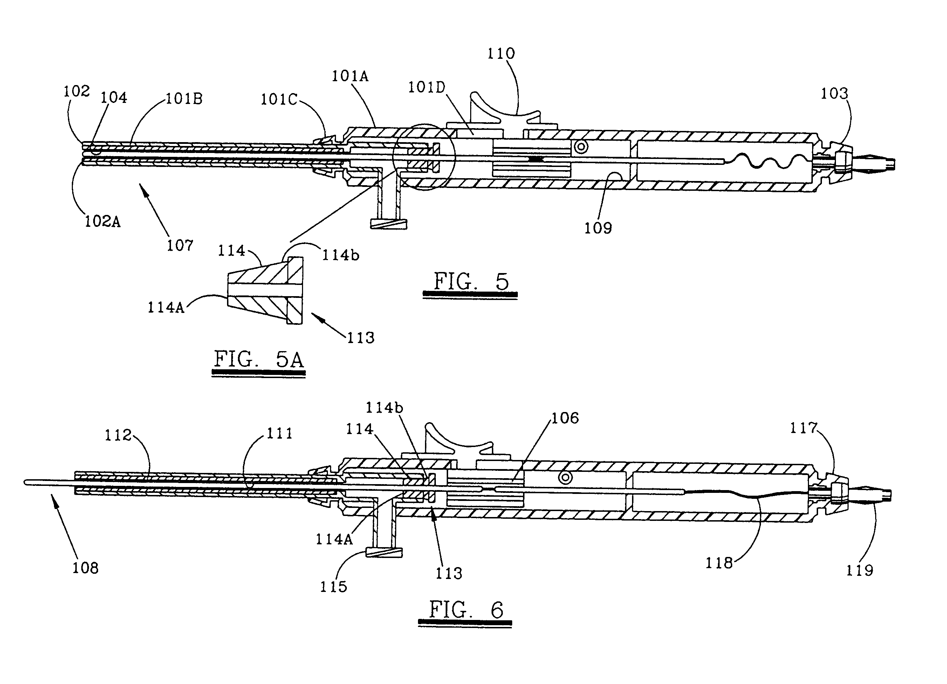

Now referring to Figs. 5 and 6, the electrosurgical instrument and probe 100 has a elongate body formed by an enlarged body member IOIA extending into a smaller diameter elongate body member IOIB. It will be appreciated that the elongate body members IOIA, IOIB, may actually be provided in one piece or may be secured, one to another, through securing head IOIC, as shown in Fig. 5 and Fig. 6.

The elongate body IOIA, IOIB, has a forward end 102 which is extended through the trocar 200 and within the abdominal wall W, as shown in Fig. 2. The forward end 102 has an opening 102A for selective receipt therethrough of the outer-most portion of an instrument head 105, particularly as shown in Fig. 4.

As shown in Fig. 5, the electrosurgical instrument and probe 100 is positioned in its rearward-most retractable position 107.

An opening IOID is provided, preferably in the form of a slot, within the elongate body member IOIA, for receipt of a thumb-activated shifting means 110 extending through the opening IOIC to a carriage 106 which is contoured to the inner diameter of the elongate body IOIA, such that the interior of the body member IOIA defines a slidable path 109 for the carriage 106.

An electrode 104 is secured within the body members IOIA, IOIB and is attached to electric conduit 118 extending to an outer cap 117 on the elongate body member lOlAand to an electrical receptacle 119 for receipt of an electrical connection (not shown) extending to an electric energy source (not shown).

The electrode 104 is secured within the carriage 106, such that as the carriage 106 slides longitudinally relative to the elongate body member IOIA, the electrode 104 will move to extend or retract the instrument head 105 from the opening 102A.

Also, as shown in Figs. 5 and 6, insulation means 112 are provided between the electrode 104 and the elongate body member IOIB to insulate the operator and/or patient from the elctrosurgical instrument, to prevent "arcing" and electric shock caused thereby. Such insulation means may be provided in the form of known materials, such as a plastic shield, or the like. Alternatively, the body member IOIB itself may be made of plastic or electric insulation means.

Interior of the elongate body member 1O1A, 101B, is provide a fluid passageway 111 extending from the opening 102A, through the body members 1O1A, 1O1B, to a port 115, which receives a fluid transmission conduit (not shown) such as for irrigation or suction purposes.

As shown in Figs. 5 and 6, the elongate body member 101 B extends through the connection 101 C and into the elongate body member 1O1A. The rearward-most open end of the elongate body member 101B within the member 101A is sealed by sealing means 113 through which is received the electrode 104. Preferably, the sealing means 113 is provided in the form of a conically shaped elastomeric element 114 having a smaller outer diameter 114A facing the opening 102A. The smaller outer diameter 114A extends to a larger outer diameter portion 114B facing the rearward-most end 103 of the apparatus 100. In such form, fluid pressure within the fluid passageway 111 will act upon the conical sealing means 113 to enhance the sealing engagement relative to the electrode 104, as fluid pressure is increased, thus further assuring the sealing integrity of the sealing means 113 to the electrode 104, to further assure abatement of fluid transmission thereacross at the rearward most end of the elongate body member 101 B.

Now referring to Figs. 1 and 2, the trocar 200 for use with the electrosurgical instrument has a housing 201 through which is disposed an opening 203 receiving a thumb applied lever 202 moveable between a forward and a rearward position for manipulating the trocar outer sleeve 208 thereon from retracted position (Fig. 1) to expanded position (Fig. 2) when the trocar 200 is introduced within the abdominal wall Wand into the abdominal cavity AC. A gear 207 is provided interior of lever 202 for companion inner engagement with gears 206 defined around the outer sleeve 208.

A rearward opening 205 is disposed within the housing 201 of the trocar 200 with a series of seal elements 204 being provided which, when the electrosurgical instrument and probe 100 are inserted within the trocar 200, sealingly engage around the elongate body member 101 B of the instrument and probe 100.

When it is desired to activate the trocar 200 to assure that it is lockingly secured within the abdominal wall W to thereby resist retracting movements, the lever 202 is manipulated to inner engage the gear members 206, 207, to cause relative movement between the outer sleeve 208 and inner sleeve 209 to expand the expanding member 212 to resist withdrawal of the trocar 200 from within the abdominal wall W. The inner sleeve 209 and the outer sleeve 208 are secured at securement 211 at the inner-most end of the trocar 200.

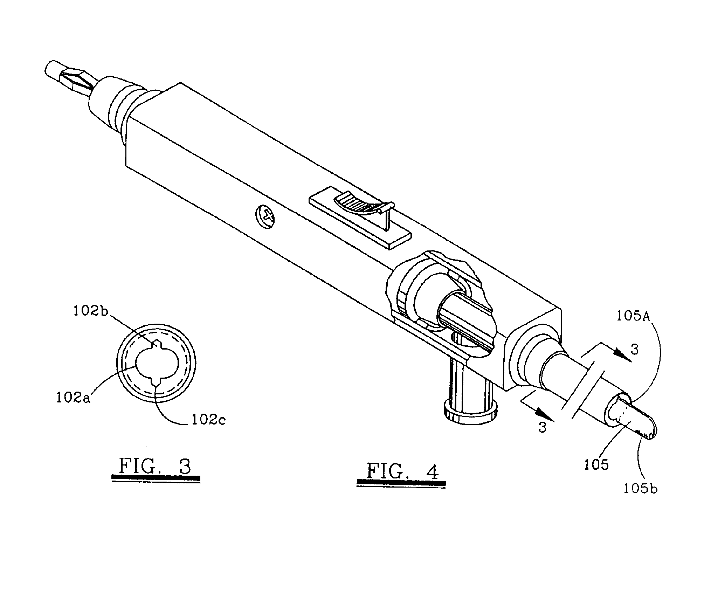

Now referring to Figs. 3 and 4, the instrument head 105 may be secured to the electrode 104 in any known fashion, such as by welding, extrusion, selectively removable securement, or the like. The instrument head 105 may actually be a profiled portion of the electrode 104. In any event, as shown, the instrument head 105 has an upper shoulder 105A and a lower shoulder 105B which are selectively moveable from within the opening 102A when the instrument 100 is in the retracted position to a position in which the instrument head 105 actually protrudes through opening 102A, as shown in Fig. 4.

Slots 102B, 102C, are provided at the opening 102A on the forward end 102 of the elongate body member 101 B for companion interengagement with the respective upper shoulder 105A and lower shoulder 105B of the instrument head 105, to prevent relative rotational movements of the instrument head 105 relative to the elongate body member 101 B.

As stated previously, the electrosurgical instrument and probe 100 may be introduced into the trocar 200 prior to the trocar 200 and instrument probe 100 being inserted into the abdominal wall W through the incision. Most conventional applications will dictate the introduction of the trocar 200 through the incision and into the abdominal wall W for positioning within the abdominal cavity AC prior to insertion of the device 100. Thereafter, the lever 202 is manipulated to position the trocar200 in expanded position as shown in Fig. 2. Thereafter, the hand H of the operator, such as the surgeon, is placed upon the elongate body member 1 01A with the thumb T in position on the shifting means 110, as shown in Fig. 1. The device 100 is introduced through the opening 205 of the trocar 200 and positioned as shown in Fig. 1, or Fig. 2.

When it is desired to move the electrode 104 from the retracted position to the expanded position, the thumb T is applied to the shifting means 110 to slidably manipulate same along exterior of the exterior of the elongate housing IOIA, such that the associated carriage 106 moves along the slidable path 109 with in the body member 1 01A to move the electrode 104 to the expanded position.

Now, the instrument head 105 will pass through the opening 102A and extend exterior of the forward end 102. Electric power may now be supplied to the device 100, either by switch activation, or by other known means.

After the electrosurgical procedure is completed, the shifting means 110 may be manipulated by placing the thumb T of the operator thereon and retracting the same relative to the elongate body member 1O1A, such that the carriage 106 pulls the electrode 104 toward the rearward end 103 of the apparatus 100, thus causing the instrument head 105 to pass through the opening 102A and be completely housed within the elongate body member 101B.

Now, the apparatus 100 may be utilized as a probe without accidental shocking, burning, or other misuse of the instrument head 105 therein. The probing function may be accomplished by using the forward blunt end 102 of the elongate body member 101B.

Of course, the electrode 104 may be selectively moved, again, and repeatedly, from the rearward position (Fig. 5) to the forward (Fig. 6) to repeat the electrosurgical procedure within the abdomen, as required.

Irrigation and/or suction through the apparatus 100 is accomplished by passage of fluid, either liquid or gas, through the port 115 and within the fluid passageway 111 and out, or in, the opening 102A and through the abdominal wall AW as required, and in known fashion. When the fluid passing through the fluid passageway 111 is liquid, such as for irrigation purposes, the pressure on the fluid as transmitted through the apparatus 100 is defined across the sealing means 113 initially at the smaller outer diameter end portion 114A, extending to the larger outer diameter end portion 114B. As such pressure is increased, the conically shaped seal 114 will increase its sealing engagement on the electrode 104 to enhance seal integrity there between.

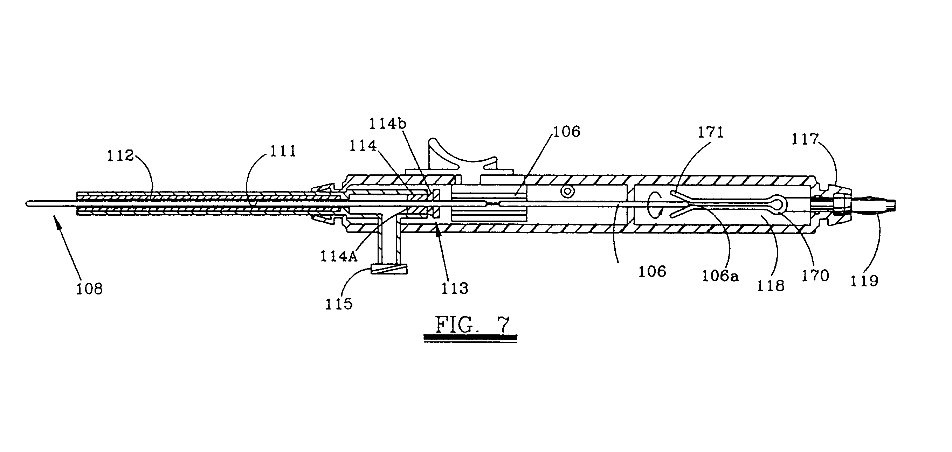

Now referring to Fig. 7, an alternate embodiment is illustrated. Like parts have been numbered as in Figs. 1 through 6. In the embodiment shown in Fig. 7, a hair pin-shaped slide connection 170 is disposed for receipt of the end 106a of the electrode 106 through open end 171 and between its parallel arms. Accordingly, by provision of the slide connection 170, the electrode is permitted to rotate relatively to the other parts. Additionally, the end 106a may move, just slightly, forwardly or rearwardly, to accommodate additional retraction of the electrode 106. By providing this slide connection 170, the electrode 106 may rotate, such that the head 105 is aligned at any angle desired. The connector 170 may be rotatable and extend through the housing through manipulation of part 119. Alternatively, the connector may be fixed and the electrode rotated either manually by hand, or, during surgery, by means of a grasper which would be inserted into the abdomen through another trocar. The electrode would be viewed through an endoscope to observe the grasping step. At any rate, it is of benefit to have an electrical connection to the electrode which would permit manipulation of the electrode tip which could take one of many geometric shapes. Having the ability to move this shape during surgery is of benefit.

## Claims

1. A laparoscopic instrument, combining an electrosurgical device and a probe, introducible into a trocar assembly by an operator and into the abdominal cavity of a patient, the instrument comprising an elongate body (101A,101 B) having forward and rearward ends; electrode means (104) housed within the body for supplying an electrical or radio-frequency signal extending substantially through the body from near the rearward end to near the forward end thereof; means (110) on the body and extending to the electrode means for selective positioning of the electrode means between rearward-most retracted and forward-most extended positions; an instrument head (105) secured to the electrode means near the forward end of the body and selectively extendable through the forward end when the electrode means is in the extended position and selectively retracted within the body when the electrode means is in the retracted position to place the instrument head completely within the elongate body, whereby when in the retracted position, the forward end of the instrument may function as a surgical probe.

2. An instrument according to claim 1, wherein the selective positioning means comprises carriage means (106) within the body and extending to the electrode means (104) for selective positioning thereof between rearward-most retracted and forward-most extended positions along a slidable path within the body relative to the forward end; and shifting means (110) disposed on the body and operatively attached to the carriage means for selectively moving the electrode means between retracted and extended positions.

3. An instrument according to claim 1 or claim 2, further comprising a fluid passageway (111) disposed in the instrument and extending from the forward end of the body for transmitting suction or irrigation fluids between the abdominal cavity and the apparatus.

4. An instrument according to claim 3, wherein the fluid passageway (111) is disposed within the body and concentric to the electrode means.

5. An instrument according to claim 4, wherein the electrode means (104) is disposed within the fluid passageway (111).

6. An instrument according to any one of the preceding claims, further including insulation means to electrically insulate the operator and the patient from the electrosurgical device.

7. An instrument according to any one of the preceding claims, further including sealing means (113) disposed within the body for sealing between the body and the electrode means to prevent fluid transmission there across.

8. An instrument according to claim 7, wherein the sealing means including a conically shaped elastomeric element (114) disposed around the electrode means, the conically shaped elastomeric element having a smaller outer diameter end facing toward the forward end and extending to a larger outer diameter end facing toward the rearward end.

9. An instrument according to claim 7 or claim 8, further including a port (115) extending through the body and into the passageway, the port being toward the forward end of the body relative to the sealing means, the port receiving one of a liquid or gaseous transmitting conduit, the pressure of the fluid through the port and the passageway enhancing the sealing engagement of the sealing means between the electrode means and the body.

10. An instrument according to any one of the preceding claims, wherein an opening (102a) at the forward end of the body has means (102b) therein for receiving the instrument head (105) therethrough and for preventing rotation of the instrument head relative to the elongate body.

11. An instrument according to any one of the preceding claims, further including means for enabling rotation of the electrode means and/or head relatively to the body.

12. A combination laparoscopic trocar assembly, comprising a trocar sleeve (200) having a first end which is arranged to extend into an abdominal cavity (AC) through a laparoscopic incision in the abdominal wall (AW), the first end of the sleeve having a first external dimension passage through the incision; means (202,207) for expanding a portion (212) of the first end of the sleeve within the abdomen so that the external dimension of the first end of the sleeve within the abdomen is expanded to a larger, second external dimension, the first end of the sleeve abutting the inner abdominal wall about the incision when expanded to the second external dimension to resist withdrawal of the trocar assembly from the abdominal cavity; a passageway through the assembly and interior of the sleeve; and, within the passageway, a laparoscopic instrument (100) according to any one of the preceding claims.
